# EUROPÄISCHE PATENTSCHRIFT

(11) **EP 3 180 615 B1**
(45) Veröffentlichungstag und Bekanntmachung des Hinweises auf die Patenterteilung: **30.01.2019**
(21) Anmeldenummer: 15760363.0
(22) Anmeldetag: 10.07.2015
(51) Int. Cl.: G01N 33/53

(54) **NACHWEISVERFAHREN UNTER VERWENDUNG VON REKOMBINANTEN LEBENDEN ZELLEN ZUM NACHWEIS VON XENOBIOTISCHEN STOFFEN SOWIE ANORDNUNG ZUR DURCHFÜHRUNG DES NACHWEISVERFAHRENS**
DETECTION METHOD USING RECOMBINANT LIVING CELLS FOR DETECTING XENOBIOTIC SUBSTANCES AND ARRANGEMENT FOR PERFORMING THE DETECTION METHOD
PROCÉDÉ DE DÉTECTION UTILISANT DES CELLULES VIVANTES RECOMBINANTES POUR LA DÉTECTION DE SUBSTANCES XÉNOBIOTIQUES ET AGENCEMENT POUR LA RÉALISATION DU PROCÉDÉ DE DÉTECTION

(30) Priorität: 13.08.2014 DE 102014012130
(43) Veröffentlichungstag der Anmeldung: 21.06.2017
(73) Patentinhaber: Alfred-Wegener-Institut Helmholtz-Zentrum für Polar- und Meeresforschung, 27570 Bremerhaven (DE)
(72) Erfinder: BICKMEYER, Ulf-Georg, 27572 Bremerhaven (DE)
(86) Internationale Anmeldenummer: PCT/DE2015/000352
(87) Internationale Veröffentlichungsnummer: WO 2016/023530

(56) Entgegenhaltungen:
- DE-T2- 69 635 355
- DE-T2- 69 824 763
- US-A1- 2004 241 832
- J A MCLACHLAN: "Functional toxicology: a new approach to detect biologically active xenobiotics", ENVIRONMENTAL HEALTH PERSPECTIVES., Bd. 101, Nr. 5, 1. Oktober 1993 (1993-10-01), Seiten 386-387, XP055221580, US ISSN: 0091-6765, DOI: 10.1289/ehp.93101386
- IWAKI TOMOKO ET AL: "A survey of all 11 ABC transporters in fission yeast: two novel ABC transporters are required for red pigment accumulation in a Schizosaccharomyces pombe adenine biosynthetic mutant.", MICROBIOLOGY (READING, ENGLAND) AUG 2006, Bd. 152, Nr. Pt 8, August 2006 (2006-08), Seiten 2309-2321, XP002748180, ISSN: 1350-0872, DOI: 10.1099/mic.0.28952-0

## Beschreibung

### Bezeichnung

Nachweisverfahren unter Verwendung von rekombinanten lebenden Zellen zum Nachweis von xenobiotischen Stoffen sowie Anordnung zur Durchführung des Nachweisverfahrens.

### Beschreibung

Die Erfindung bezieht sich auf ein Nachweisverfahren unter Verwendung von rekombinanten lebenden Zellen zum Nachweis von xenobiotischen Stoffen. Dabei werden rekombinante Zellen mit mindestens einem Gensequenzabschnitt aᵢ eingesetzt, wobei der Gensequenzabschnitt aᵢ durch Fusion mit mindestens einem Markerprotein-Gen mᵢ rekombinant verändert ist. Die Auswirkungen des xenobiotischen Stoffs auf das exprimierte Gen-Fusionsprodukt aᵢ-mᵢ werden über das enthaltene Markerprotein-Gen mᵢ detektiert. Weiterhin bezieht sich die Erfindung auf eine Anordnung zur Durchführung des Nachweisverfahrens.

Xenobiotische Stoffe (Fremdstoffe) sind chemische Verbindungen, die dem biologischen Stoffkreislauf eines Organismus fremd sind. Sie enthalten teilweise Strukturelemente, die in dieser Form nicht oder nur äußerst selten in Naturstoffen vorkommen. In der Natur sind Xenobiotika häufig anthropogenen Ursprungs, beispielsweise künstliche Pflanzenschutzmittel, halogenierte Kohlenwasserstoffe oder Kunststoffe. Viele dieser Stoffe sind biologisch nur schwer oder gar nicht abbaubar. Xenobiotika können positiv, gar nicht oder aber auch schädlich auf die Umwelt und Organismen einwirken. Ein schädlicher toxischer Effekt hängt neben der Aufnahme auch von der biologischen Abbaubarkeit sowie von der Anreicherung in bestimmten Organismen oder Teilen von Organismen ab.

Xenobiotische Stoffe können in fester, flüssiger oder gasförmiger Form in Systemen ebenfalls aller drei Aggregatszustände auftreten. Insbesondere in aquatischen Systemen, beispielsweise Flüsse, Seen oder Meere, treten oft anthropogene und biogene xenobiotische Stoffe auf, deren genaue chemische Natur nicht bekannt ist und/oder deren Konzentration so gering ist, dass sie keine toxikologische Relevanz besitzen. Trotzdem ist ihre Kenntnis oft hilfreich, wenn nicht sogar zwingend erforderlich. Die bekannte chemische Analytik mit ihren toxikologischen Tests mit Modellorganismen und Zellen und im weitesten Sinne die bekannten Ökotests funktionieren in der Regel nur bei höheren Konzentrationen und liefern auch nur wenig Hinweise auf die Substanzklasse der Chemikalien. Somit ergibt sich insbesondere bei aquatischen Systemen die besondere Problemstellung, wie solche Substanzen schnell und zuverlässig detektiert werden können.

### Stand der Technik

Der der Erfindung nächstliegende Stand der Technik ist aus der DE 698 24 763 T2 bekannt. Es wird ein Nachweisverfahren beschrieben, das die Identifikation eines toxischen Stoffs, der direkt oder indirekt die Translokation eines biologisch aktiven Polypeptids beeinflusst, ermöglicht. Dabei ist das biologisch aktive Polypeptid ein Polypeptid, das intrazelluläre Abläufe beeinflusst, nachdem ein intrazellulärer Signaltransduktionsweg aktiviert wurde. Der intrazelluläre Signaltransduktionsweg umfasst eine enzymatische Reaktion und ist ein koordinierter intrazellulärer Ablauf, in dem eine lebende Zelle ein externes oder internes Signal in zelluläre Antworten umwandelt. Es werden eine oder mehrere Zellen kultiviert, die eine stabile Expression einer Nukleinsäure zeigen, die für ein Hybridpolypeptid kodiert. Dabei umfasst das Hybridpolypeptid ein Fluorophor, insbesondere ein Grün-Fluoreszenz-Protein GFP, das von einer Nukleinsäure kodiert wird, die mit dem biologisch aktiven Polypeptid oder einem Teil davon verbunden ist. Die Zellen werden mit einem für die biologische Funktion oder biologische Wirkung zu screenenden toxischen Stoff inkubiert. Es wird das Licht, das von dem Fluorophor in den inkubierten Zellen emittiert wird, in Hinblick auf Veränderungen detektiert. Dabei handelt es sich um Ortsveränderungen, d.h. mittels der Fluoreszenzmarkierungen werden Ortsveränderungen des Hybridpolypeptids in der Zelle ortsaufgelöst detektiert. Solche detektierten Ortsveränderungen zeigen eine Veränderung der Translokation des biologisch aktiven Polypeptids in den inkubierten Zellen und damit deren Wechselwirkung mit speziellen toxischen Stoffen an. Das bekannte Nachweisverfahren untersucht zelluläre Systeme, in denen durch den Einfluss eines toxischen Stoffes eine intrazelluläre Translokation eines biologisch aktiven Polypeptids erfolgt und stellt eine schnelle und reproduzierbare Quantifizierung der Translokationsantworten bereit. Dadurch wird es möglich, zwischen den Einflüssen auf zelluläre Systeme und deren Translokationsantworten sinnvolle Rückschlüsse zu ziehen. Zwingend ist hierfür aber eine stabile Expression der Hybrid erforderlich, um eine solche Translokation mit dem bekannten Nachweisverfahren detektieren zu können. Zusammenfassend gesehen ist das Nachweisverfahren gemäß der DE 698 24 763 T2 somit nur auf translozierende Stoffe, die eine intrazelluläre Antwort auslösen, anwendbar und führt ein ortsaufgelöste Detektion von Markerproteinen durch, die auf einer stabilen Expression der eingesetzten Fusionsproteine basiert.

Ähnliches ist aus der DE 696 35 355 T2 bekannt. Auch hier werden mit GFP markierte Hybridpolypeptide erzeugt, um die biologische Aktivität einer Probe zu charakterisieren. Es erfolgt jedoch keine Lokalisation der Fluoreszenz, sondern eine Messung von deren Intensität und ein Vergleich mit Normalwerten.

Auch in der WO 95/07463 A1 werden eine Zelle, die fähig ist, GFP zu exprimieren, und ein Verfahren zum Nachweisen eines interessierenden Proteins in einer Zelle beschrieben, das auf dem Einführen eines Gens in eine Zelle, das einen Gensequenzabschnitt aufweist, die das interessierende Protein mit einem Gensequenzabschnitt verbindet, der ein GFP kodiert, basiert, sodass das durch das Gen hergestellte Protein das interessierende Protein fusioniert an das GFP aufweist. Nach dem Kultivieren der Zellen wird dann die Fluoreszenz in der Zelle lokalisiert, wodurch auch das interessierende Protein in der Zelle lokalisiert wird. Auch hierbei werden also wieder intrazelluläre Prozesse durch Fluoreszenzdetektion lokalisiert. Die WO 95/07463 A1 beschreibt ferner Zellen, die für den Nachweis von Molekülen, wie Hormone oder Schwermetalle, in einer biologischen Sonde eingesetzt werden. Dabei können lebende Organismen diese Zellen aufweisen. Das regulatorische Element des Gens, das durch das interessierende Molekül beeinflusst wird, ist operativ mit einem GFP verbunden. Bei dem regulatorischen Element kann es sich um einen Promotor handeln, der zu einem Gen gehört, das für die Zelllebensfähigkeit verantwortlich ist. Durch die Anwesenheit der Moleküle wird das regulatorische Element beeinflusst, was wiederum die Expression des GFP beeinflusst. Auf diese Weise wird das Gen kodierend für GFP als Reportergen in einer Zelle verwendet, die zum Überwachen der Anwesenheit einer spezifischen molekularen Identität konstruiert ist. Mit diesem bekannten Verfahren können somit beispielsweise Stresseinwirkungen auf den lebenden Organismus durch verstärkte Fluoreszenz angezeigt werden.

Aus der oben genannten DE 698 24 763 T2 ist es implizit bekannt, dass mit einem Markerprotein fusionierte Funktionsproteine im Fusionsprotein in ihrer Funktion nicht beeinträchtigt sind und unverändert exprimiert werden können. Analoges ist auch aus der **Veröffentlichung** "Structural requirements for the apical sorting of multidrug resistance protein 2 (ABCC2)" von A.T. Nies et al. (in Eur. J. Bio chem. 269, 1866-1876 (2002)) bekannt. Hier wird aufgezeigt, dass prinzipiell GFP-Ankopplungen an MRP-Transporterproteine funktionieren und die Transporterfunktionen nicht gestört werden. Die Transporterproteine arbeiten auch mit der Markierung einwandfrei. Ziel auch dieser Veröffentlichung ist aber, die Lokalisation der Transporter nachzuweisen und denjenigen Anteil der Transporter festzustellen, der für die Lokalisation an bestimmte Orte zuständig ist. Für eine Ankopplung von GFPs an menschliche MDR-Proteine ist Ähnliches aus der **Veröffentlichung** "Sensitive and Specific Fluorescent Probes for Functional Analysis of the Three Major Types of Mammalian ABC Transporters" von I.V. Lebedeva et al. (in PLoS ONE July 2011, Vol. 6, Issue7, e22429, pp 1 bis 12). Hier werden die GFPs als Indikatoren für MDRs in lebende Zellen in einer durchflusszytrometrischen Messung genutzt.

ABC-Transporter bilden eine große Familie von Membranproteinen, die als gemeinsames Strukturelement eine ATP (Adenosintriphosphat)-bindende Kassette (ABC Akronym von ATP Binding Cassette) besitzen und chemische Substrate aktiv über eine Zellmembran transportieren. Chemische Substanzen können, auch wenn sie in toxikologisch irrelevanten Konzentrationen auftreten, eine Reaktion in lebenden Organismen und deren Zellen hervorrufen, indem sie das erste zelluläre Verteidigungssystem, zu dem die ABC-Transporter gehören, aktivieren oder hochregulieren. Lebende Organismen können somit einen Schutzmechanismus aktivieren, der bei Bedarf unter Energieverbrauch Fremdstoffe aus dem Zellinneren und dann auch aus ganzen Geweben entfernen kann. Am Transport beteiligt sind vor allem Multiple Drug Resistance Proteine (MDR) und Multidrug Resistance-Related Proteine (MRP). Mit diesen Bezeichnungen wird das Phänomen beschrieben, dass Zellen eine Resistenz gegenüber speziellen Stoffen haben bzw. entwickeln.

Über die MDR- und MRP-Transporterproteine beim Plattwurm *Macrostomum lignano* im Zusammenhang mit einer Inkubation mit dem fluoreszierenden Farbstoff Ageladine A wird ausführlich in der **Veröffentlichung** "Reporter Dyes Demonstrate Functional Expression of Multidrug Resistance Proteins in the Marine Flatworm Macrostomum lignano: The Sponge-Derived Dye Ageladine A Is Not a Substrate of these Transporters" von K. Tietje et al. (in Mar. Drugs 2013, 11, 3951-3969) berichtet.

Das grün fluoreszierende Protein (Abkürzung GFP; engl. green fluorescent protein) ist ein erstmals 1961 von Osamu Shimomura beschriebenes Protein aus der Qualle *Aequorea victoria,* das bei Anregung mit blauem oder ultraviolettem Licht grün fluoresziert. Modifizierte Varianten des GFP weisen andere Farbspektren auf. Seine unschätzbare Bedeutung in der Biologie, insbesondere der Zellbiologie, liegt in der Möglichkeit, GFP mit beliebigen anderen Proteinen Gen-spezifisch zu fusionieren. Durch die Fluoreszenz des GFP kann so die räumliche und zeitliche Verteilung des anderen Proteins in lebenden Zellen, Geweben oder Organismen direkt beobachtet werden. Über die GFPs und ihr Wesen wird ausführlich in der **Veröffentlichung** "Nobel lecture: constructing and exploiting the fluorescent protein paintbox" von R.T. Tsien berichtet (in Integr. Biol. 2010, 2, 77-93). Neuere Varianten des GFPs werden beispielsweise in der DE 696 04 298 T2 beschrieben.

Weiterhin wird in der **Veröffentlichung** "Functional toxicology: a new approach to detect biologically active xenobiotics" von J.A. McLachlan (Environmental Health Perspectives, Bd. 101, Nr. 5, 1. Oktober 1992, Seiten 386-387, DOI: 10.1289/ehp.931001386) die Detektion von biologisch aktiven Xenobiotika mit Hilfe von funktioneller Toxikologie offenbart. Zellen werden mit molekularen Konstrukten transfiziert, die einen spezifischen Rezeptor und ein Reportergen für diesen Rezeptor beinhalten. So ist es möglich, Chemikalien von unbekannter Biologie oder Toxikologie zu screenen und ihre Funktion zu determinieren. Eine integrale Messung findet nicht statt.

Die US 2004/241832 A1 offenbart einen Zellkulturbeobachtungsapparat, der einen Habitatbehälter mit bestimmten Kulturbedingungen, ein Abbildungssystem und eine Analysesektion beinhaltet. Lumineszenz wird gemessen. Autofluoreszenz kann auch gemessen werden. Es wird aber kein Behälter offenbart, der beispielsweise flüssigkeitsdurchlässig ist.

Die **Veröffentlichung** "A survey of all 11 ABC transporters in fission yeast: two novel ABC transporters are required for red pigment accumulation in a Schizosaccharomyces pombe adenini biosynthetic mutant" von Iwaki Tomoko et al. (Micobiology (Reading, England) Aug 2006, Bd. 152, Nr. Pt8, August 2006, Seiten 2309-2321 (ISSN: 1350-0872)) beschreibt die Lokalisierung GFPgebundener ABC-Transporterproteine in Zellen von Spalthefe. Ein Kit wird nicht offenbart. Eine integrale Messung findet nicht statt.

### Aufgabenstellung

Ausgehend von dem bekannten Nachweisverfahren als nächstliegendem Stand der Technik gemäß der oben näher erläuterten DE 698 24 763 T2 ist die Aufgabe für die vorliegende Erfindung darin zu sehen, eine Weiterentwicklung anzugeben, mit der die Anwesenheit von möglichst vielen xenobiotischen Stoffen zuverlässig, einfach und schnell nachgewiesen werden kann. Dies soll auch dann möglich sein, wenn die genaue chemische Natur der Substanzen nicht bekannt ist oder wenn die Substanzen in solchen Konzentrationen auftreten, dass sie keine toxikologische Relevanz besitzen. Weiterhin soll eine Anordnung zur Durchführung des Nachweisverfahrens angegeben werden, die dessen Umsetzung in die reale Praxis besonders einfach und zuverlässig und darüber hinaus autark macht. Dabei soll ein Testkit zum Einsatz kommen, das sich idealerweise mit der Anordnung ergänzt und die Anwendbarkeit des Nachweisverfahrens durch Zurverfügungstellung unterschiedlicher rekombinanter Zellen besonders vielseitig macht. Diese Aufgaben werden von dem Verfahrensanspruch und den beiden Erzeugnisansprüchen gelöst. Vorteilhafte Modifikationen der Lösungen werden in den jeweiligen Unteransprüchen aufgezeigt und im Folgenden im Zusammenhang mit der Erfindung näher erläutert.

Erfindungsgemäß wird beansprucht ein Nachweisverfahren unter Verwendung von rekombinanten lebenden Zellen zum Nachweis von solchen xenobiotischen Stoffen, die bei Inkontaktbringung mit den lebenden Zellen über zumindest einen in den lebenden Zellen aktiven Promotor eine Aktivierung oder Hochregulierung der Expression von mindestens einem Gensequenzabschnitt aᵢ, der für mindestens ein Funktionsprotein Aᵢ mit bekannter Funktion kodiert, bewirken, wobei der Gensequenzabschnitt aᵢ durch Fusion mit mindestens einem Markerprotein-Gen mᵢ, das für mindestens ein die Kodierung des Funktionsproteins Aᵢ nicht beeinflussendes Markerprotein Mᵢ mit bekannten Markereigenschaften kodiert, rekombinant verändert ist und das Gen-Fusionskonstrukt aᵢ-mᵢ für ein Fusionsprotein Aᵢ-Mᵢ aus dem mindestens einen Funktionsprotein Aᵢ und dem mindestens einen Markerprotein Mᵢ kodiert und wobei ein verstärktes Auftreten des mindestens einen Markerproteins Mᵢ als Zellreaktion durch eine Detektion seiner Markereigenschaften integral über die Fläche erfasst wird. Dabei bezeichnet i den Laufindex mit i=1 bis n für verschiedene Gensequenzabschnitte aᵢ, Funktionsproteine Aᵢ, Markerprotein-Gene mᵢ und Markerproteine Mᵢ.

Das erfindungsgemäße Verfahren kann als eine sehr effiziente Vorgehensweise zum Testen oder Entdecken des Einflusses einer xenobiotischen Substanz auf einen physiologischen Prozess verwendet werden, insbesondere in Verbindung mit dem Testen von Stoffen auf Toxizität. Es unterscheidet sich von dem bekannten Nachweisverfahren vor allem auch dadurch, dass die Grundlage der Messung eine dynamisch veränderliche Expression des Fusionsproteins Aᵢ-Mᵢ bildet. Eine vermehrte Bildung des Markerproteins Mᵢ wird integral über eine Fläche detektiert und zeigt zuverlässig an, dass auch das Funktionsprotein Aᵢ vermehrt gebildet wird. Dabei wird durch Fremdstoffe in den lebenden Zellen viel häufiger eine stärkere Expression von einzelnen Funktionsproteinen hervorgerufen als eine Translokation von Proteinen, bei der es sich um einen sehr viel komplexeren Zellvorgang handelt. Der Fokus der Erfindung liegt damit auf der dynamischen Expression als Sensorsignal, während die Expression bei dem bekannten Nachweisverfahren als konstant angenommen wird und eine wesentliche Voraussetzung darstellt. Die Beeinflussung der Expression von unterschiedlichen Proteinen in lebenden Zellen im Sinne von Veränderung aufgrund von xenobiotischen Stoffen liegt der Erfindung als grundlegendes Wirkprinzip zugrunde. Die dynamische Expression wird bei der Erfindung aufgefasst als ein Fremdstoffsignal, das die zelluläre Antwort auf eine Kontamination ist. Bei der Erfindung werden die lebenden transgenen Zellen als Sensoren eingesetzt, wobei die zellulären Vorgänge und deren Umsetzung in eine Zellantwort im Sinne einer Nachrichtenverarbeitung und -übertragung im Nachweisverfahren genutzt werden. Bei der Erfindung handelt es sich um ein Nachweisverfahren unter Anwendung von Sensortechnik auf der Basis von lebenden Zellen, das den entscheidenden Schritt aus dem theoretischen Forschungslabor in die praktische aquatische Realität vollzieht.

Das erfindungsgemäße Nachweisverfahren bietet eine über die Fläche integrale und nicht - wie der Stand der Technik - eine ortsaufgelöste Analyse der Zellantworten an, wobei die integrale Detektion schnell, einfach und zuverlässig durchführbar ist. Lange Inkubationszeiten der transgenen Zellen entfallen, sodass das erfindungsgemäße Nachweisverfahren auch für schnelle Durchflussmessungen bestens geeignet ist. Auch ist eine integrale Detektion sehr viel einfacher durchzuführen als eine ortsaufgelöste Detektion, die immer auch voraussetzt, dass die transgenen Zellen einzeln beobachtbar sind. Hierfür sind zeitaufwändige Vereinzelungsvorgänge erforderlich. Bei der integralen Detektion im erfindungsgemäßen Nachweisverfahren kann die Zellantwort der Zellen im Verband einfach beobachtet und quantitativ ausgewertet werden. Auch eine Automatisierung des Nachweisverfahrens in einem autarken Messsystem ist somit problemlos durchführbar.

Bei den für das erfindungsgemäße Nachweisverfahren genutzten Signalwegen zwischen dem Fremdstoff und der transgenen Zelle wirkt der nachzuweisende Stoff auf entsprechende Promotoren in der rekombinanten lebenden Zelle ein und aktiviert oder hochreguliert deren Genexpression, die die Ausprägung bzw. den Aktivitätszustand eines oder mehrerer Gene beschreibt. Als Promotor wird in der Genetik eine Nukleotidsequenz auf der DNA bezeichnet, die die regulierte Expression eines Gens ermöglicht. Der Promotor ist ein essenzieller Bestandteil eines Gens. Er liegt am 5'-Ende des Gens (Kopfende) und somit vor dem RNA-kodierenden Bereich. Die wichtigste Eigenschaft eines Promotors ist die spezifische Wechselwirkung mit bestimmten DNA-bindenden Proteinen, welche den Start der Transkription des Gens durch die RNA-Polymerase vermitteln (Transkriptionsfaktoren).

Mit dem Nachweisverfahren nach der Erfindung können grundsätzlich alle xenobiotischen Stoffe nachgewiesen werden, die bei Inkontaktbringung mit nicht rekombinanten lebenden Zellen über zumindest einen in den lebenden Zellen aktiven Promotor eine Aktivierung oder Hochregulierung der Expression von mindestens einem Gensequenzabschnitt bewirken. Die Anwendung des Nachweisverfahrens nach der Erfindung kann Ergebnisse erbringen, die einen rein informellen Charakter haben, beispielsweise kann es von Interesse sein, an welchen Orten in unterschiedlichen Systemen bestimmte, insbesondere anthropogene Fremdstoffe angereichert werden. Auch kann es von Interesse sein, zu kontrollieren, ob in einem natürlichen oder technischen System überhaupt xenobiotische Stoffe auftreten, auch wenn diese zunächst unbekannter Natur sind. Ein anderer Nutzen des Nachweisverfahrens nach der Erfindung besteht in der Warnung des Menschen vor dem Auftreten von für ihn schädlichen oder sogar toxischen Stoffen. Bevorzugt und vorteilhaft ist daher bei dem Nachweisverfahren nach der Erfindung vorgesehen, dass xenobiotische Stoffe, die toxischer und/oder unbekannter Natur sind und/oder in Konzentrationen ohne toxikologische Relevanz auftreten, nachweisbar sind. Besonders vorteilhaft und bevorzugt sind mit dem Nachweisverfahren nach der Erfindung xenobiotische Stoffe in der Umgebung von technischen Anlagen nachweisbar. Das Nachweisverfahren nach der Erfindung eignet sich daher besonders gut für den Betrieb von Warnanlagen in der Nähe solcher technischer Anlagen. Ein Betrieb in der Nähe von Raffinerien, chemischen Anlagen und Kraftwerken ist besonders sinnvoll, um toxische Stoffe, wie beispielsweise Schwermetalle oder halogenierte Kohlenwasserstoffe, schnell und zuverlässig nachweisen zu können. Je nach Art der xenobiotischen Stoffe werden dabei unterschiedliche Proteine in den rekombinanten lebenden Zellen aktiviert und durch deren gentechnische Markierung nachweisbar.

Voraussetzung für die Anwendbarkeit des Nachweisverfahrens nach der Erfindung ist die Ansprache zumindest eines Promotors in den lebenden Zellen durch den nachzuweisenden xenobiotischen Stoff, wobei der angesprochene Promotor als Reaktion dann einen speziellen Gensequenzabschnitt aktiviert oder hochreguliert. Durch die Inkontaktbringung mit dem xenobiotischen Stoff wird eine Zellreaktion aktiviert. Da es vorstellbar ist, dass zu ein und demselben Zeitpunkt verschiedene xenobiotische Stoffe auftreten und diese unterschiedliche Promotoren in den Zellen als Reaktion ansprechen, sodass dann verschiedene Gensequenzabschnitte aktiviert oder hochreguliert werden. Deshalb ist es vorteilhaft und bevorzugt, wenn verschiedene Gensequenzabschnitte aᵢ, die für unterschiedliche Funktionsproteine Aᵢ kodieren, mit Markerprotein-Genen mᵢ, die für Markerproteine Mᵢ mit unterschiedlichen Markereigenschaften kodieren, rekombinant verändert sind und die Gen-Fusionskonstrukte aᵢ-mᵢ für verschiedene Fusionsproteine Aᵢ-Mᵢ kodieren, wobei über die Detektion der unterschiedlichen Markereigenschaften auf die vermehrte Kodierung für die entsprechenden Funktionsproteine Aᵢ rückgeschlossen wird. Über die entsprechend vermehrt erzeugten Funktionsproteine Aᵢ kann dann auf Art und Charakter der verschiedenen xenobiotischen Stoffe rückgeschlossen werden. Dabei können die kontaminierenden xenobiotischen Stoffe in Abhängigkeit von ihren chemischen Eigenschaften unterschiedliche Zellreaktionen auslösen.

Eine besonders wichtige und häufig auftretende Zellreaktion ist die Aktivierung des zelleigenen Abwehrsystems. Besonders vorteilhaft und bevorzugt ist es daher, wenn der mindestens eine durch xenobiotische Stoffe aktivierte oder in seiner Expression hochregulierte Gensequenzabschnitt aᵢ ein solcher ist, der für die Abwehr- und Detoxifizierungsmechanismen der lebenden Zellen kodiert, wobei das kodierte Funktionsprotein Aᵢ in den Fusionsproteinen Aᵢ-Mᵢ ein Mitglied der Familie der ABC-Transporterproteine ist, deren Funktion der Fremdstoff-Membrantransport ist. Die nachzuweisenden xenobiotischen Stoffe regen also das erste zelluläre Verteidigungssystem der rekombinanten Zellen an und führen zu den detektierbaren Zellreaktionen. Da dieses zelleigene Verteidigungssystem sehr breit aufgestellt ist, können mit den Nachweisverfahren nach der Erfindung sehr viele xenobiotische Stoffe nachgewiesen werden. Insbesondere können sicher und zuverlässig sehr viele schädliche toxische Stoffe - auch in geringsten Konzentrationen - nachgewiesen werden, weil das zelleigene Abwehrsystem - ohne große Unterschiede zwischen den verschiedenen Spenderorganismen - konsequenterweise für die Abwehr genau solcher Stoffe konzipiert ist.

Insbesondere ist es bevorzugt und vorteilhaft, wenn der Gensequenzabschnitt aᵢ für ein Funktionsprotein Aᵢ in den Fusionsproteinen Aᵢ-Mᵢ in Form eines Multidrug Resistance Proteins (MDR-Transporterprotein) und/oder eines Multidrug Resistance-Associated Proteins (MRP-Transporterprotein) aus der Familie der ABC-Transporterproteine kodiert. Hierbei handelt es sich um diejenigen Transporterproteine aus der großen Familie der ABC-Transporter, die am häufigsten auftreten und am intensivsten am zelleigenen Abwehrsystem beteiligt sind. Bereits weiter oben wurde ausgeführt, dass bei dem Nachweisverfahren nach der Erfindung verschiedene Gensequenzabschnitte aᵢ, die für unterschiedliche Funktionsproteine Aᵢ kodieren, mit unterschiedlichen Markerprotein-Genen mᵢ, die für unterschiedliche Markerproteine Mᵢ mit verschiedenen Markereigenschaften (siehe auch unten) kodieren, fusioniert sein können. Mit dem Nachweisverfahren nach der Erfindung kann also eine erhöhte Expression von MDR-Transporterproteinen und von MRP-Transporterproteinen zuverlässig detektiert werden. Daher ist es möglich, dass bei dem Nachweisverfahren nach der Erfindung bei einem verstärkten Auftreten von MDR-Transporterproteinen als Zellreaktion auf einen xenobiotischen Stoff mit ungeladenen Molekülen mit Kettenlängen in derselben Größenordnung und bei einem verstärkten Auftreten von MRP-Transporterproteinen als Zellreaktion auf einen xenobiotischen Stoff mit geladenen Molekülen mit Kettenlängen zwischen 100 Da (Einheit Dalton) und 8 kDa, bevorzugt zwischen 1 und 3 kDa, besonders bevorzugt zwischen 1,5 und 2,5 kDa, beispielsweise bis zu 2 kDa, rückgeschlossen wird. Durch eine solche Zuordnung kann der xenobiotische Stoff, insbesondere wenn es sich um einen unbekannten toxischen Stoff handelt, in seinem Ursprung bereits eingegrenzt werden.

Markerproteine können die unterschiedlichsten Markereigenschaften chemischer und physikalischer Natur aufweisen, die entsprechend detektierbar sind. Beispielsweise können Markerproteine den pH-Wert ihrer Umgebung messbar verändern. Einfacher ist aber die optische Detektion der Markerproteine, beispielsweise die Detektion der Größe. Die einfachste Detektion ist jedoch die Farberkennung, insbesondere wenn das Markerprotein selbsttätig oder unter Anregung fluoresziert. Daher ist es beim Nachweisverfahren nach der Erfindung vorteilhaft und bevorzugt, wenn Markerproteine Mᵢ mit Fluoreszenz als Markereigenschaft eingesetzt werden, wobei zumindest die Fluoreszenzwellenlänge und/oder die Fluoreszenzintensität detektiert werden. Dabei können bevorzugt und vorteilhaft Markerproteine Mᵢ aus der Familie der Green Fluorescent Proteine (GFP) oder ihrer fluoreszierenden Homologen oder von Derivaten oder von Mutanten dieser GFPs eingesetzt werden. Die GFPs sind gut erforscht und handhabbar und stehen in vielen Farbvarianten zur Verfügung.

Das modifizierte Nachweisverfahren nach der Erfindung kann somit eine auf den GFP und deren farblichen Abwandlungen basierende Farbmarkierung von unterschiedlichen ABC-Transportern in lebenden transgenen rekombinanten Zellen umfassen, um in Abhängigkeit von deren dynamischem Expressionsgrad einen Nachweis der Existenz von anthropogenen und biogenen Fremdstoffen im Wasser zu erbringen. Das aus Medusen der Art Aequoria stammende fluoreszierende Protein GFP und dessen Abwandlungen werden in das Genom der lebenden Zellen eingeschleust und die Proteine zusammen mit den ABC-Transportern exprimiert. Wenn die ABC-Transporter verstärkt exprimiert werden, wird auch das GFP verstärkt exprimiert und somit wird die Fluoreszenz erhöht. Es werden rekombinante Zellen bzw. Organismen (siehe unten) erzeugt, die auf Umweltveränderungen mit erhöhter Expression von MDR/MRP reagieren und dadurch die Fluoreszenz im gewählten GFP-Bereich erhöhen.

Das Nachweisverfahren nach der Erfindung basiert auf den Reaktionen rekombinanter lebender Zellen auf deren Kontamination mit xenobiotischen Stoffen. Grundsätzlich kann die Kontamination der Zellen mit einem xenobiotischen Stoff in allen drei Aggregatszuständen erfolgen. Neben der Kontamination der lebenden Zellen mit einem festen oder gasförmigen Fremdstoff kann der nachzuweisende Fremdstoff insbesondere auch in einer wässrigen Lösung verteilt oder aufgelöst sein, sodass eine Kontamination der lebenden Zellen in wässriger Phase auftritt. Neben einer Kontamination in einem Gewässer sind hier auch Anwendungsfälle im medizinischen Bereich, beispielsweise im Zusammenhang mit Blut, denkbar. Bei der wässrigen Kontamination können grundsätzlich lebende Zellen bzw. ganze Zellkulturen in entsprechenden Nährlösungen gehalten werden. Vorteilhaft und bevorzugt ist es dabei, wenn die lebende Zellen von aquatischen Organismen, der die lebenden Zellen bereits aufweist, oder von einem nicht rekombinanten aquatischen Mutterorganismus stammen, wobei es sich dabei bevorzugt um ein Krebstier, ein Nesseltier, eine Seeanemone, ein Stacheltier, einen Schwamm oder einen Rund- oder Plattwurm, besonders bevorzugt um den Plattwurm *Macrostonum lignano,* handeln kann. Im zweiten Fall werden dem Mutterorganismus lebende Zellen entnommen und im Labor im Sinne der Erfindung gentechnisch verändert.

Wird ein lebender aquatischer Organismus verwendet, der die rekombinanten Zellen bereits enthält, ist es vorteilhaft und bevorzugt, wenn zumindest ein vollständiger lebender aquatischer Organismus, der die lebenden Zellen aufweist, eingesetzt wird. Dabei kann es sich bei dem gentechnisch veränderten Organismus wiederum bevorzugt um ein Krebstier, ein Nesseltier, eine Seeanemone, ein Stacheltier, einen Schwamm oder einen Rund- oder Plattwurm, besonders bevorzugt um den Plattwurm *Macrostonum lignano,* handeln. Die Haltung solcher einfachen aquatischen Organismen ist relativ einfach. Von Vorteil ist es dabei, wenn die eingesetzten aquatischen Organismen genetisch zumindest teilweise bereits entschlüsselt sind (was bei dem Plattwurm *Macrostonum lignano* der Fall ist, hier sind bereits viele EST - Expressed Sequence Tags - bekannt), sodass die rekombinante Veränderung der speziellen Gensequenzabschnitte aᵢ mit den Markerprotein-Genen mᵢ von Fachleuten routinemäßig vorgenommen werden kann. Die lebenden Zellen mit dem Fusionskonstrukt aᵢ-mᵢ können sich überall in dem kontaminierten Organismus ansiedeln und vermehren, insbesondere auch auf der Oberfläche bzw. der Haut der Organismen. Dort können markierende Fluoreszenzerscheinungen unter Kontamination bezüglich Farbe und Intensität dann einfach detektiert werden. Um aber die Fluoreszenzmarker im gesamten Organismus zuverlässig detektieren zu können, ist es besonders vorteilhaft und bevorzugt, wenn zumindest ein Organismus eingesetzt wird, das zumindest teilweise transparent ist. Der aquatische Organismus in Form des Plattwurms *Macrostonum lignano* ist weitgehend transparent, sodass auch Fluoreszenzerscheinungen in seinem Inneren gut detektierbar sind. Bei einer Kontamination der rekombinante Zellen mit festen oder gasförmigen Fremdstoffen können ebenfalls ganze Organismen eingesetzt werden, die dann entsprechend detektierbare Reaktionen beispielsweise auf der Haut oder anderen äußeren Bereichen oder in der Lunge oder anderen inneren Organen zeigen.

Bei der Detektion von Emissionen ist bezüglich deren Farben und Intensitäten natürlich eine Detektion bevorzugt und vorteilhaft, die als optische Detektion in automatisierter Weise durchgeführt wird. Bei anderen zu detektierenden Markereigenschaften werden andere Detektionsprinzipien angewendet. Beispielsweise können pH-Wertänderungen mit dem fluoreszierenden Farbstoff Ageladine A, insbesondere auch in transparenten Organismen, sehr gut nachgewiesen werden. Detektionen, die auf elektrischen Größen, wie Strom und Spannung, basieren, sind ebenfalls anwendbar, wenn die Markereigenschaften einen Einfluss auf Strom und Spannung haben. Eine optische Detektion ist aber berührungsfrei und kann als einfache Beobachtung auch besonders gut automatisiert durchgeführt werden.

Geeignete Anordnungen zur Durchführung des Nachweisverfahrens nach der Erfindung richten sich nach der Art der Kontamination, insbesondere nach deren Aggregatzustand. Bei festen und gasförmigen Kontamination kann ein ganzer Organismus in Luft gehalten werden, Zellkulturen sind in wässrigen Lösungen vorzuhalten. Im Nachfolgenden wird eine beanspruchte Anordnung bei einer flüssigen, insbesondere wässrigen Kontamination aufgezeigt. Die beanspruchte erfindungsgemäße Anordnung zur Durchführung des Nachweisverfahrens nach der Erfindung unter Verwendung von lebenden Zellen aquatischer Organismen ist gekennzeichnet durch einen Habitatbehälter zur Lebendhaltung zumindest eiern Zellkultur oder zumindest eines vollständigen lebenden aquatischen Organismus in einem aquatischen System mit einer wasserdurchlässigen Wandung, die einen Wasseraustausch mit dem Wasser des umgebenden aquatischen Systems zulässt und eine verschließbare Öffnung zum Einsetzen und Entfernen der lebenden Zellkultur oder des lebenden aquatischen Organismus aufweist, durch ein Futtersystem zur automatischen Fütterung der lebenden Zellkultur oder des lebenden aquatischen Organismus und durch ein automatisches Detektionssystem mit einem Detektor zur Überwachung der Markereigenschaften der fusionierten Markerproteine in den Zellen der lebenden Zellkultur oder des lebenden aquatischen Organismus sowie einer Datenstation zumindest zur Speicherung der detektierten Daten und einer Sendestation zur Übermittlung der detektierten Daten an eine externe Station. Bei einer optischen Detektion wird bevorzugt und vorteilhaft entsprechend ein optisches Detektionssystem, insbesondere zur Fluoreszenzdetektion bei fluoreszierenden Markereigenschaften, eingesetzt. Die eingesetzten gentechnisch veränderten Zellen oder kompletten aquatischen Organismen werden aber zu keinem Zeitpunkt freigesetzt, sondern leben in den verschlossenen Behältern, die jedoch jederzeit den Wasseraustausch ermöglichen.

Zusätzlich kann noch bevorzugt und vorteilhaft eine optische Signaleinrichtung vorgesehen sein, die in Abhängigkeit von den detektierten Daten automatisch auslösbar ist und im Falle einer Auslösung am Ort des Habitatbehälters sichtbar ist und/oder eine Überwachungseinrichtung am Habitatbehälter zur Überwachung des Lebendzustands der eingesetzten aquatischen Organismen. Solche Systeme sind dann besonders gut als autarke Frühwarnsysteme in der Nähe von technischen Anlagen mit einem zu erwartenden Ausstoß an xenobiotischen Stoffen einzusetzen, um sich dort aufhaltende Menschen einfach warnen zu können. Diese Systeme selbst arbeiten biologisch völlig unbedenklich und umweltschonend.

Weiterhin ist ein Testkit zur Durchführung des Nachweisverfahrens mit einem Mittel auf der Basis von rekombinante lebenden Zellen zur Durchführung des Nachweisverfahrens zu Verfügung stellbar, wobei das Mittel eine Ausbildung in Form von lebenden Zellen mit mindestens einem Gen-Fusionskonstrukt aᵢ-mᵢ aus einem Gensequenzabschnitt aᵢ, der für mindestens ein Funktionsprotein Aᵢ aus der Familie der ABC-Transporterproteine kodiert, und einem Markerprotein-Gen mᵢ, das für mindestens ein die Kodierung des Funktionsproteins Aᵢ nicht beeinflussendes Markerprotein Mᵢ aus der Familie der Green Fluorescent Proteine kodiert, wobei das Gen-Fusionskonstrukt aᵢ-mᵢ für ein Fusionsprotein Aᵢ-Mᵢ aus dem Funktionsprotein Aᵢ und dem Markerprotein Mᵢ kodiert, zeigt. Transporterproteine aus dem Abwehrsystem einer lebenden Zelle, die mit einem fluoreszierenden GFP-Markerprotein rekombinant verändert und markiert sind, werden bislang nicht als Sensoren zur Detektion von Schadstoffen eingesetzt. Dabei können die eingesetzten lebenden Zellen erzeugt werden durch eine rekombinante Herleitung von einem nicht rekombinante aquatischen Mutterorganismus, bevorzugt von einem Krebstier, einem Nesseltier, einer Seeanemone, einem Stacheltier, einem Schwamm oder einem Rund- oder Plattwurm, besonders bevorzugt von dem Plattwurm *Macrostonum lignano.* Weiterhin können die lebenden Zellen aus nicht rekombinanten Zellkulturen oder nicht rekombinanten immortalisierten Zelllinien rekombinant hergeleitet sein. Solche Zellkulturen und Zelllinien können in öffentlich zugänglichen Sammlung von Mikroorganismen und Zellkulturen hinterlegt sein. Gleiches gilt auch für Zellkulturen und Zelllinien mit den rekombinanten Zellen. Nähere Einzelheiten zu der Erfindung sind dem nachfolgenden Ausführungsbeispiel zu entnehmen.

### Ausführungsbeispiel

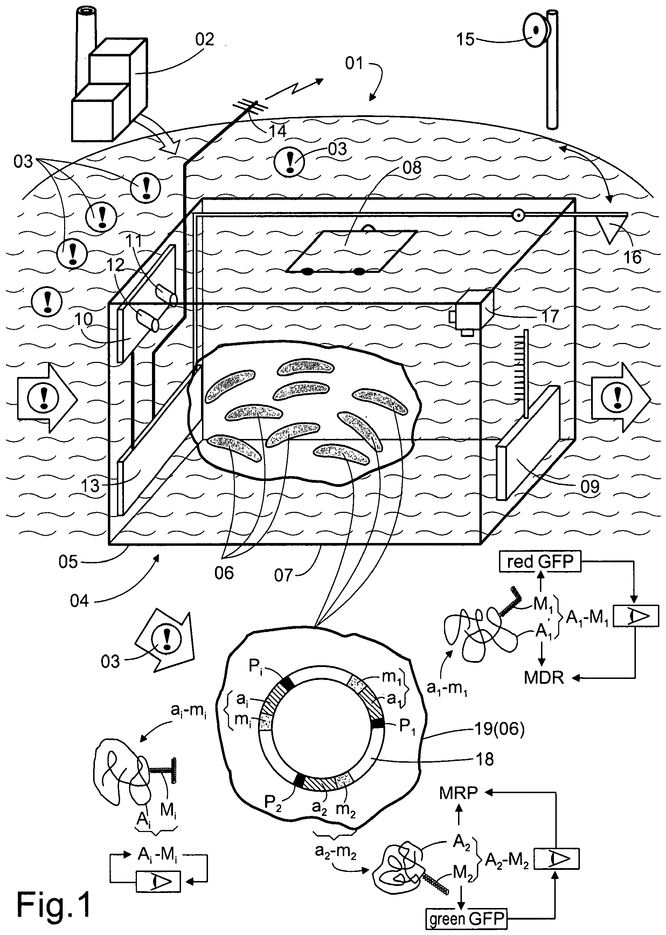

Das Nachweisverfahren und die Anordnung zur Durchführung des Nachweisverfahrens nach der Erfindung und ihre vorteilhaften Modifikationen werden anhand der schematischen, nicht maßstäblichen **Figur 1** zum besseren Verständnis der Erfindung an einem Ausführungsbeispiel noch weitergehend erläutert. Dabei könnte das Ausführungsbeispiel bezeichnet werden mit "Farbkodierung von xenobiotischen chemischen Strukturen durch fluoreszierende marine Organismen" (engl. "color coding of xenobiotic chemical structures by fluorescent marine organisms").

In der **Figur 1** ist ein aquatisches System **01** dargestellt, im dargestellten Beispiel eine Meeresbucht. Am Rande der Bucht befindet sich eine technische Anlage **02**, im dargestellten Beispiel eine Chemieanlage. Von der technischen Anlage **02** werden xenobiotische Stoffe **03** (Sinnbild Ausrufezeichen) in das aquatische System **01** eingeleitet. In der Bucht baden Menschen. Eine Frühwarnung vor schädlichen Stoffen ist somit also äußerst sinnvoll.

Im Vordergrund ist eine Anordnung **04** zur Durchführung des Nachweisverfahrens nach der Erfindung dargestellt. Gezeigt ist ein Habitatbehälter **05** zur Lebendhaltung einer Mehrzahl von aquatischen Organismus **06** (im aufgebrochenen Ausschnitt), im gezeigten Beispiel handelt es sich um Plattwürmer der Gattung *Macrostonum lignano,* die in dem aquatischen System **01** angeordnet sind. Diese Plattwürmer sind weitgehend transparent und lassen eine Detektion von Fluoreszenzerscheinungen in ihrem Inneren problemlos zu. Der Habitatbehälter **05** weist eine wasserdurchlässige Wandung **07** auf. Diese ermöglicht einen ungestörten Wasseraustausch mit dem Wasser des umgebenden aquatischen Systems **01**. In der Wandung **07** befinden sich eine verschließbare Öffnung **08** zum Einsetzen und Entfernen der aquatischen Organismen **06** und ein Futtersystem **09** zur automatischen Fütterung der aquatischen Organismen **06**. Weiterhin befindet sich im Habitatbehälter **05** ein automatisches Detektionssystem **10** mit einer Lichtquelle **11** zur Bestrahlung der aquatischen Organismen **06** und einem Detektor **12**, im gezeigten Ausführungsbeispiel einem optischen Detektor, zur Überwachung der aquatischen Organismen **06** im Sinne einer Detektion ihrer Fluoreszenzemission. Weiterhin sind eine Datenstation **13**, die eine batteriegestützte oder externe Spannungsversorgung aufweist, zumindest zur Speicherung der detektierten Daten und eine Sendestation **14** zur Übermittlung der detektierten Daten an eine externe Empfangsstation **15** vorgesehen. Zusätzlich ist am Habitatbehälter **05** noch eine optische Signaleinrichtung **16** angeordnet, die in Abhängigkeit von den detektierten Daten automatisch auslösbar ist und im Falle einer Auslösung am Ort des Habitatbehälters **05** als Warnung vor eine Kontamination durch aufgetretene xenobiotische, insbesondere toxische Stoffe sichtbar ist. Schließlich ist noch eine Überwachungseinrichtung **17** am Habitatbehälter **05** zur Überwachung des Lebendzustands der eingesetzten aquatischen Organismen **06** vorgesehen. Hierbei kann es sich beispielsweise um eine Videokamera handeln.

Die gentechnische Veränderung der lebenden Zellen der aquatischen Organismen **06**, um diese für einen Einsatz in dem Nachweisverfahren nach der Erfindung einsetzen zu können, sind in dem unteren vergrößerten Ausschnitt in der **Figur 1** dargestellt. Gezeigt ist in einer rekombinanten lebenden Zelle **19** ein Plasmid **18** mit einem Gensequenzabschnitt **a₁**, der für ein Funktionsprotein **A₁**, im gewählten Ausführungsbeispiel ein MDR-Transporterprotein, kodiert, und mit einem Gensequenzabschnitt **a₂**, der für ein Funktionsprotein **A₂**, im gewählten Ausführungsbeispiel ein MRP-Transporterprotein, kodiert. Beide Transporterproteine stammen aus der Familie der ABC-Transporterproteine. Der Gensequenzabschnitt **aᵢ** wird von einem Promotor **P₁** aktiviert bzw. hochreguliert, der Gensequenzabschnitt **a₂** wird von dem Promotor **P₂** aktiviert bzw. hochreguliert. Der Gensequenzabschnitt **a₁** ist mit einer Marker-Gensequenz **m1** rekombinant verändert, der Gensequenzabschnitt **a₂** ist mit einer Marker-Gensequenz **m₂** rekombinant verändert. Die Gensequenz **m₁** kodiert im gewählten Ausführungsbeispiel für ein grün fluoreszierendes Markerprotein **M₁**, die Marker-Gensequenz **m₂** kodiert im gewählten Ausführungsbeispiel für ein rot fluoreszierendes Markerprotein **m₂**. Beide Markerproteine stammen aus der Familie der GFP. Das Gen-Fusionskonstrukt aus dem Gensequenzabschnitt **a₁** und der Marker-Gensequenz **m₁** ist mit **a₁-m₁** bezeichnet. Es kodiert für das markierte Fusionsprotein **A₁-M₁**. Das Gen-Fusionskonstrukt aus dem Gensequenzabschnitt **a2** und der Marker-Gensequenz **m₂** ist mit **a₂-m₂** bezeichnet. Es kodiert für das markierte Fusionsprotein **A₂-M₂**. Die Bezeichnungen mit dem Laufindex i zeigen an, dass noch weitere Gensequenzabschnitte **aᵢ** durch weitere Marker **mᵢ** rekombinant verändert sein können, die dann für andere Funktionsproteine **Aᵢ** und andere Markerproteine **Mᵢ** mit anderen Markereigenschaften in anderen Fusionskonstrukten **Ai-Mi** kodieren.

Im gezeigten Ausführungsbeispiel wird bei einer Inkontaktbringung des Plasmids **18** des aquatischen Organismus **06** mit dem xenobiotischen Stoff **03** das zelleigene Abwehrsystem über den Promotor **P₁** aktiviert. Dadurch werden vermehrt MDR-Proteine **A₁** und damit Markerproteine **M₁** erzeugt, die bei Anregung grün fluoreszieren. Mittels des automatischen Detektionssystems **10** wird die auftretende oder verstärkte grüne Fluoreszenzemission erkannt. Die Daten werden an die externe Empfangsstation **15** übermittelt und ausgewertet. Gleichzeitig wird die optische Signaleinrichtung **16** ausgelöst und die Menschen, die sich in und in der Nähe des aquatischen Systems **01** aufhalten, zuverlässig und frühzeitig gewarnt.

Mit dem Nachweisverfahren nach der Erfindung auf Basis rekombinanter Gensequenzen im Genom einer gentechnisch veränderten Zelle, die bei Inkontaktbringung mit einem xenobiotischen Stoff, eine Zellantwort in Form einer dynamischen Genexpression generiert, kann somit ein relativ einfaches und zuverlässiges Warnsystem für den Menschen aufgebaut werden. Insbesondere können auch solche Kontaminationen detektiert werden, bei denen aufgrund zu geringer Konzentrationen andere Nachweisverfahren versagen. Zusammen mit der Anordnung und dem Testkit zur Durchführung des Nachweisverfahrens nach der Erfindung, das insbesondere auf der Hälterung von gentechnisch veränderten, lebenden vollständigen aquatischen Organismen in einem aquatischen System beruht, kann ein bevorzugtes komplexes System für die Praxis angeboten werden, dass unterschiedliche xenobiotische Stoffe in den unterschiedlichsten Situationen zuverlässig detektiert und davor warnt.

### Bezugszeichenliste

- 01: aquatisches System
- 02: technische Anlage
- 03: xenobiotischer Stoff
- 04: Anordnung zur Durchführung des Nachweisverfahrens
- 05: Habitatbehälter
- 06: vollständiger lebender aquatischer Organismus (gentechnisch verändert), der lebende Zellen 19 aufweist
- 07: wasserdurchlässige Wandung
- 08: verschließbare Öffnung
- 09: Futtersystem
- 10: automatisches Detektionssystem
- 11: Lichtquelle
- 12: Detektor
- 13: Datenstation
- 14: Sendestation
- 15: externe Empfangsstation
- 16: optische Signaleinrichtung
- 17: Überwachungseinrichtung
- 18: Plasmid
- 19: rekombinante lebende Zelle

- aᵢ: Gensequenzabschnitt, i=1..n
- mᵢ: Marker-Gensequenz
- aᵢ-mᵢ: Gen-Fusionskonstrukt
- Aᵢ: Funktionsprotein
- Mᵢ: Markerprotein
- Aᵢ-Mᵢ: Fusionsprotein
- Pᵢ: Promotor

## Patentansprüche

1. Nachweisverfahren unter Verwendung von rekombinanten lebenden Zellen (19) zum Nachweis von solchen xenobiotischen Stoffen (03), die bei Inkontaktbringung mit den lebenden Zellen (19) über zumindest einen in den lebenden Zellen (19) aktiven Promotor Pᵢ eine Aktivierung oder Hochregulierung der Expression von mindestens einem Gensequenzabschnitt aᵢ, der für mindestens ein Funktionsprotein Aᵢ mit bekannter Funktion kodiert, bewirken, wobei der Gensequenzabschnitt aᵢ durch Fusion mit mindestens einem Markerprotein-Gen mᵢ, das für mindestens ein die Kodierung des Funktionsproteins Aᵢ nicht beeinflussendes Markerprotein Mᵢ mit bekannten Markereigenschaften kodiert, rekombinant verändert ist und das Gen-Fusionskonstrukt aᵢ-mᵢ für ein Fusionsprotein Aᵢ-Mᵢ aus dem mindestens einen Funktionsprotein Aᵢ und dem mindestens einen Markerprotein Mᵢ kodiert und wobei ein verstärktes Auftreten des mindestens einen Markerproteins Mᵢ als Zellreaktion durch eine Detektion seiner Markereigenschaften integral erfasst wird.

2. Nachweisverfahren nach Anspruch 1,
**dadurch gekennzeichnet, dass** xenobiotische Stoffe (03), die toxischer und/oder unbekannter Natur sind und/oder in Konzentrationen ohne toxikologische Relevanz auftreten, nachweisbar sind.

3. Nachweisverfahren nach Anspruch 1 oder 2,
**dadurch gekennzeichnet, dass** xenobiotische Stoffe (03) in der Umgebung von technischen Anlagen (02) und/oder in aquatischen Systemen (01) nachweisbar sind.

4. Nachweisverfahren nach einem der vorangehenden Ansprüche,
**dadurch gekennzeichnet, dass** verschiedene Gensequenzabschnitte aᵢ, die für unterschiedliche Funktionsproteine Aᵢ kodieren, mit Markerprotein-Genen mᵢ, die für Markerproteine Mᵢ mit unterschiedlichen Markereigenschaften kodieren, rekombinant verändert sind und die Gen-Fusionskonstrukte aᵢ-mᵢ für verschiedene Fusionsproteine Aᵢ-Mᵢ kodieren, wobei über die Detektion der unterschiedlichen Markereigenschaften auf die vermehrte Kodierung für die entsprechenden Funktionsproteine Aᵢ rückgeschlossen wird.

5. Nachweisverfahren nach einem der vorangehenden Ansprüche,
**dadurch gekennzeichnet, dass** der mindestens eine durch einen xenobiotischen Stoff (03) aktivierte oder in seiner Expression hochregulierte Gensequenzabschnitt aᵢ ein solcher ist, der für die Abwehr- und Detoxifizierungsmechanismen der lebenden Zellen (19) kodiert, wobei das kodierte Funktionsprotein Aᵢ in den Fusionsproteinen Aᵢ-Mᵢ ein Mitglied der Familie der ABC-Transporterproteine ist, deren Funktion der Fremdstoff-Membrantransport ist.

6. Nachweisverfahren nach Anspruch 5,
**dadurch gekennzeichnet, dass** der Gensequenzabschnitt aᵢ für ein Funktionsprotein Aᵢ in den Fusionsproteinen Aᵢ-Mᵢ in Form eines Multidrug Resistance Proteins (MDR-Transporterprotein) und/oder eines Multidrug Resistance-Associated Proteins (MRP-Transporterprotein) aus der Familie der ABC-Transporterproteine kodiert.

7. Nachweisverfahren nach Anspruch 6,
**dadurch gekennzeichnet, dass** bei einem verstärkten Auftreten von MDR-Transporterproteinen als Zellreaktion auf einen xenobiotischen Stoff (03) mit ungeladenen Molekülen mit Kettenlängen in derselben Größenordnung und bei einem verstärkten Auftreten von MRP-Transporterproteinen als Zellreaktion auf einen xenobiotischen Stoff mit geladenen Molekülen mit Kettenlängen zwischen 100 Da und 8 kDa, bevorzugt zwischen 1 und 3 kDa, besonders bevorzugt zwischen 1,5 und 2,5 kDa, rückgeschlossen wird.

8. Nachweisverfahren nach einem der vorangehenden Ansprüche,
**dadurch gekennzeichnet, dass** Markerproteine Mᵢ mit Fluoreszenz als Markereigenschaft eingesetzt werden, wobei zumindest die Fluoreszenzwellenlänge und/oder die Fluoreszenzintensität detektiert werden.

9. Nachweisverfahren nach Anspruch 8,
**dadurch gekennzeichnet, dass** Markerproteine Mᵢ aus der Familie der Green Fluorescent Proteine (GFP) oder ihrer fluoreszierenden Homologen oder von Derivaten oder von Mutanten dieser GFPs eingesetzt werden.

10. Nachweisverfahren nach einem der vorangehenden Ansprüche,
**dadurch gekennzeichnet, dass** die lebenden Zellen (19) von einem aquatischen Organismus (06), der die lebenden Zellen (19) bereits aufweist, oder von einem nicht rekombinanten aquatischen Mutterorganismus stammen, wobei es sich dabei bevorzugt um ein Krebstier, ein Nesseltier, eine Seeanemone, ein Stacheltier, einen Schwamm oder einen Rund- oder Plattwurm, besonders bevorzugt um den Plattwurm *Macrostonum lignano,* handeln kann.

11. Nachweisverfahren nach einem der vorangehenden Ansprüche,
**dadurch gekennzeichnet, dass** zumindest ein vollständiger lebender aquatischer Organismus (06), der die lebenden Zellen (19) aufweist, eingesetzt wird, wobei es sich dabei bevorzugt um ein Krebstier, ein Nesseltier, eine Seeanemone, ein Stacheltier, einen Schwamm oder einen Rund- oder Plattwurm, besonders bevorzugt um den Plattwurm *Macrostonum lignano,* handeln kann.

12. Nachweisverfahren nach Anspruch 10 oder 11,
**dadurch gekennzeichnet, dass** der eingesetzte aquatische Organismus (06) zumindest teilweise transparent ist.

13. Nachweisverfahren nach einem der vorangehenden Ansprüche,
**dadurch gekennzeichnet, dass** die Detektion als optische Detektion in automatisierter Weise durchgeführt wird.

14. Anordnung (04) zur Durchführung des Nachweisverfahrens nach einem der vorangehenden Ansprüche,
**gekennzeichnet durch** einen Habitatbehälter (05) zur Lebendhaltung zumindest einer Zellkultur mit den lebenden Zellen (19) oder zumindest eines vollständigen lebenden aquatischen Organismus (06), der die lebenden Zellen (19) aufweist, in einem aquatischen System (01) mit einer wasserdurchlässigen Wandung (07), die einen Wasseraustausch mit dem Wasser des umgebenden aquatischen Systems (01) zulässt und eine verschließbare Öffnung (08) zum Einsetzen und Entfernen der Zellkultur oder des aquatischen Organismus (06) aufweist, durch ein Futtersystem (09) zur automatischen Fütterung der Zellkultur oder des aquatischen Organismus (06) und durch ein automatisches Detektionssystem (10) mit einem Detektor (12), insbesondere einem optischen Detektor, zur Überwachung der Markereigenschaften der fusionierten Markerproteine in den Zellen der Zellkultur oder des aquatischen Organismus (06) sowie einer Datenstation (13) zumindest zur Speicherung der detektierten Daten und einer Sendestation (14) zur Übermittlung der detektierten Daten an eine externe Station (15).

15. Anordnung (04) nach Anspruch 14,
**gekennzeichnet durch** eine optische Signaleinrichtung (16), die in Abhängigkeit von den detektierten Daten automatisch auslösbar ist und im Falle einer Auslösung am Ort des Habitatbehälters (05) sichtbar ist und/oder eine Überwachungseinrichtung (17) am Habitatbehälter (05) zur Überwachung des Lebendzustands der eingesetzten Zellkultur oder des aquatischen Organismus (06).

## Claims

1. Detection method using recombinant living cells (19) for detecting such xenobiotic substances (03) which on being brought into contact with living cells (19) by way of at least one promotor Pᵢ active in living cells (19) bring about activation or upregulation of the expression of at least one gene sequence section aᵢ which codes for at least one functional protein Aᵢ with a known function, wherein the gene sequence section aᵢ through fusion with at least one marker protein gene mᵢ, which for at least one marker protein Mᵢ with known marker properties that does not influence the coding of the functional protein Aᵢ, is recombinantly modified and codes the gene fusion construct aᵢ-mᵢ for a fusion protein Aᵢ-Mᵢ of the at least one functional protein Aᵢ and the at least one marker protein Mᵢ and wherein a boosted presence of the at least one marker protein Mᵢ as a cell reaction is integrally recorded through detection of its marker properties.

2. Detection method according to claim 1
**characterised in that** xenobiotic substances (03) that are of a toxic and/or unknown nature and/or occur in concentrations with no toxicological relevance can be detected.

3. Detection method according to claim 1 or 2
**characterised in that** xenobiotic substances (03) in the vicinity of technical installations (02) and/or in aquatic systems (01) are detectable.

4. Detection method according to any one of the preceding claims
**characterised in that** various gene sequence sections aᵢ which code for different functional proteins Aᵢ, are recombinantly modified with marker protein genes mᵢ which code for marker proteins Mᵢ with different marker properties and code the gene fusion construct aᵢ-mᵢ for different fusion proteins Aᵢ-Mᵢ. wherein by way of detection of the different marker properties increased coding for the corresponding functional proteins Aᵢ-is deduced.

5. Detection method according to any one of the preceding claims **characterised in that** the at least one gene sequence section aᵢ activated or upregulated in its expression by a xenobiotic substance (03) is one which codes for the defence and detoxification mechanisms of the living cells (19) wherein the coded functional protein Aᵢ in the fusion proteins Aᵢ-Mᵢ is a member of the family of ABC transporter proteins, the function of which is foreign substance membrane transport.

6. Detection method according to claim 5
**characterised in that** the gene sequence section aᵢ for a functional protein Aᵢ-in the fusion proteins Aᵢ-Mᵢ codes in the form of a multidrug resistance protein (MDR transporter protein) and/or a multidrug resistance-associated protein (MRP transporter protein) from the family of ABC transporter proteins.

7. Detection method according to claim 6
**characterised in that** in the event of boosted occurrence of MDR transporter proteins as a cell reaction a xenobiotic substance (03) with uncharged molecules with chain lengths of the same size order is deduced and in the event of boosted occurrence of MRP transporter proteins as a cell reaction a xenobiotic substance with charged molecules with a chain length between 100 Da and 8 kDa, preferably between 1 and 3 kDa, particularly preferably between 1.5 and 2.5 kDa is deduced.

8. Detection method according to any one of the preceding claims **characterised in that** marker proteins Mᵢ with fluorescence as a marker property are used, wherein at least the fluorescence wavelength and/or the fluorescence intensity is detected.

9. Detection method according to claim 8
**characterised in that** marker proteins Mᵢ from the family of green fluorescent proteins (GFP) or their fluorescing homologues or of derivatives or mutants of these GFPs are used.

10. Detection method according to any one of the preceding claims
**characterised in that** the living cells (19) originate from an aquatic organism (06) that already has the living cells (19) or from a not recombinant aquatic parent organism, wherein preferably this can be a crustacean, a cnidarian, a sea anemone, an echinoderm, a sponge or a round or flat worm, particularly preferably the flat worm *Macrostonum lignano.*

11. Detection method according to any one of the preceding claims
**characterised in that** at least a complete living aquatic organism (06) which has the living cells (19) is used, wherein preferably this can be a crustacean, a cnidarian, a sea anemone, an echinoderm a sponge or a round or flat worm, particularly preferably the flat worm Macrostonum lignano.

12. Detection method according to claim 10 or 11 **characterised in that** the aquatic organism (06) used is at least partially transparent.

13. Detection method according to any one of the preceding claims
**characterised in that** detection is carried out in an automated matter as optical detection.

14. Device (04) for carrying out the detection method according to any one of the preceding claims
**characterised by** a habitat container (05) for keeping alive at least one cell culture with the living cells (19) or at least a complete living aquatic organism (06) that has the living cells (19) in an aquatic system (01) with a water-permeable wall (07) which allows an exchange of water with the water of the surrounding aquatic system (010) and a closable opening (08) for introducing and removing the cell culture of the aquatic organism (06), by feeding system (09) for the automatic feeding of the cell culture or the aquatic organism (06) and by an automatic detection system (10) with a detector (12), in particular an optical detector, for monitoring the marker properties of the fusioned marker proteins in the cells of the cell culture or the aquatic organism (06) as well as a data station (13) at least for storing the detected data and a transmitter station (14) for transmitting the detected data to an external station (15).

15. Device (04) according to claim 14
**characterised by** an optical signal device (16) which is automatically activatable depending on the detected data and in the event of activation is visible at the site of the habitat container (05) and/or a monitoring device (17) on the habitat container (05) for monitoring the state of life of the used cell culture or the aquatic organism (06).

## Revendications

1. Procédé de dépistage utilisant des cellules vivantes recombinantes (19), destiné à dépister des substances xénobiotiques (03), qui lors de leur mise en contact avec les cellules vivantes (19), par l'intermédiaire d'au moins un promoteur Pᵢ actif dans les cellules vivantes (19), provoquent une activation ou une régulation à la hausse de l'expression d'au moins un segment de séquence génétique aᵢ codant pour au moins une protéine fonctionnelle Aᵢ dont la fonction est connue, le segment de séquence génétique aᵢ codant par fusion avec au moins un gène de protéine marqueur mᵢ, codant pour au moins une protéine marqueur Mᵢ qui n'influence pas le codage de la protéine fonctionnelle Aᵢ à propriétés de marquage connues étant modifié par recombinaison et pour une protéine fusionnelle Aᵢ-Mᵢ, la construction de fusion génétique aᵢ-mᵢ codant à partir de l'au moins une protéine fonctionnelle Aᵢ et de l'au moins une protéine marqueur Mᵢ, et une recrudescence de l'au moins une protéine marqueur Mᵢ étant intégralement détectée comme une réaction cellulaire par une détection de ses propriétés de marqueur.

2. Procédé de dépistage selon la revendication 1, **caractérisé en ce que** des substances xénobiotiques (03) qui sont de nature toxique et/ou inconnue et/ou qui se présentent dans des concentrations dénuées de pertinence toxicologique sont dépistables.

3. Procédé de dépistage selon la revendication 1 ou 2, **caractérisé en ce que** des substances xénobiotiques (03) dans l'environnement d'installations techniques (02) et/ou de systèmes aquatiques (01) sont dépistables.

4. Procédé de dépistage selon l'une quelconque des revendications précédentes, **caractérisé en ce que** différents segments de séquences génétiques aᵢ, codant pour différentes protéines fonctionnelles Aᵢ sont modifiés par recombinaison avec des gènes de protéines marqueurs mᵢ, codant pour des protéines marqueurs Mᵢ disposant de différentes propriétés de marquage et les constructions de fusion génétiques aᵢ-mᵢ codant pour différentes protéines fusionnelles Aᵢ-Mᵢ, un codage multiple pour les protéines fonctionnelles Aᵢ correspondantes étant déduit par la détection des différentes propriétés de marquage.

5. Procédé de dépistage selon l'une quelconque des revendications précédentes, **caractérisé en ce que** l'au moins un segment de séquence génétique aᵢ, activé ou régulé à la hausse dans son expression par une substance xénobiotique (03) en est un du type codant pour les mécanismes immunitaires ou détoxifiants des cellules vivantes (19), la protéine fonctionnelle Aᵢ codée dans les protéines fusionnelles Aᵢ-Mᵢ étant un membre de la famille des protéines transporteuses ABC dont la fonction réside dans le transport membranaire de substances étrangères.

6. Procédé de dépistage selon la revendication 5, **caractérisé en ce que** le segment de séquence génétique aᵢ pour une protéine fonctionnelle Aᵢ dans les protéines fusionnelles Aᵢ-Mᵢ code sous la forme d'une protéine de résistance multi-médicamenteuse (protéine transporteuse MDR) et/ou d'une protéine impliquée dans la résistance multi-médicamenteuse (protéine transporteuse MPR) de la famille des protéines transporteuses ABC.

7. Procédé de dépistage selon la revendication 6, **caractérisé en ce que** lors d'une recrudescence de protéines transporteuses MDR, on déduit une réaction cellulaire à une substance xénobiotique(03) à molécules non chargées, d'une longueur de chaîne dans le même ordre de grandeur et lors d'une recrudescence de protéines transporteuses MPR, on déduit une réaction cellulaire à une substance xénobiotique à molécules chargées, d'une longueur de chaîne comprise entre 100 Da et 8 kDa, de préférence entre 1 et 3 kDa, de manière particulièrement préférentielle, entre 1,5 et 2,5 kDa.

8. Procédé de dépistage selon l'une quelconque des revendications précédentes, **caractérisé en ce qu'**on met en oeuvre des protéines marqueurs Mᵢ dotées d'une fluorescence comme propriété de marquage, au moins la longueur d'ondes de fluorescence et/ou l'intensité de fluorescence étant détectée.

9. Procédé de dépistage selon la revendication 8, **caractérisé en ce qu'**on met en oeuvre des protéines marqueurs Mᵢ de la famille des protéines vert fluorescent (GFP) ou de leurs homologues fluorescents ou de dérivés ou de mutants desdites GFP.

10. Procédé de dépistage selon l'une quelconque des revendications précédentes, **caractérisé en ce que** les cellules vivantes (19) émanent d'un organisme aquatique (06) présentant d'ores et déjà les cellules vivantes (19) ou d'un organisme aquatique mère non recombinant, pouvant s'agir de préférence à cet effet d'un crustacé, d'un cnidaire, d'une anémone des mers, d'un oursin, d'une éponge, d'un ver rond ou d'un ver plat, de préférence du ver plat *macrostonum lignano.*

11. Procédé de dépistage selon l'une quelconque des revendications précédentes, **caractérisé en ce qu'**on met en oeuvre au moins un organisme aquatique (06) vivant complet, présentant les cellules vivantes (19), pouvant s'agir de préférence à cet effet d'un crustacé, d'un cnidaire, d'une anémone des mers, d'un oursin, d'une éponge, d'un ver rond ou d'un ver plat, de préférence du ver plat *macrostonum lignano.*

12. Procédé de dépistage selon la revendication 10 ou 11, **caractérisé en ce que** l'organisme aquatique (06) mis en oeuvre est au moins partiellement transparent.

13. Procédé de dépistage selon l'une quelconque des revendications précédentes, **caractérisé en ce que** la détection est réalisée de manière automatique, sous la forme d'une détection optique.

14. Agencement (04), destiné à réaliser le procédé de dépistage selon l'une quelconque des revendications précédentes, **caractérisé par** un conteneur habitat (05), destiné à garder en vie au moins une culture cellulaire des cellules vivantes (19) ou au moins un organisme aquatique (06) vivant complet, comportant les cellules vivantes (19) dans un système aquatique (01), présentant une paroi (07) perméable à l'eau, qui admet un échange d'eau avec l'eau du système aquatique (01) environnant et une ouverture (08) obturable, pour la mise en oeuvre et le retrait de la culture cellulaire ou de l'organisme aquatique (06), par un système d'alimentation (09) destiné à l'alimentation automatique de la culture cellulaire ou de l'organisme aquatique (06) et par un système de détection (10) automatique, pourvu d'un détecteur (12), notamment d'un détecteur optique, destiné à superviser les propriétés de marquage des protéines marqueurs fusionnées dans les cellules de la culture cellulaire ou de l'organisme aquatique (06), ainsi que par un poste de données (13), destiné au moins à mémoriser les données détectées et par un poste émetteur (14), destiné à transmettre les données détectées à un poste externe (15).

15. Agencement (04) selon la revendication 14, **caractérisé par** un système de signalisation optique (16), déclenchable automatiquement en fonction des données détectées et dans le cas d'un déclenchement, visible sur le lieu du conteneur habitat (05) et/ou par un système de supervision (17) sur le conteneur habitat (05), destiné à superviser l'état vivant de la culture cellulaire ou de l'organisme aquatique (06) mis en oeuvre.
